# EUROPÄISCHE PATENTSCHRIFT

(11) **EP 0 717 617 B1**
(45) Veröffentlichungstag und Bekanntmachung des Hinweises auf die Patenterteilung: **25.10.2000**
(21) Anmeldenummer: 94926878.3
(22) Anmeldetag: 25.08.1994
(51) Int. Cl.: A61K 9/16, A61K 9/50

(54) **WIRKSTOFFE UND GAS ENTHALTENDE MIKROPARTIKEL**
ACTIVE PRINCIPLE AND GAS CONTAINING MICROPARTICLES
NOUVELLES MICROPARTICULES CONTENANT DES PRINCIPES ACTIFS ET DU GAZ

(30) Priorität: 09.09.1993 DE 4330958; 11.05.1994 DE 4416818
(43) Veröffentlichungstag der Anmeldung: 26.06.1996
(73) Patentinhaber: SCHERING AKTIENGESELLSCHAFT, 13353 Berlin (DE)
(72) Erfinder: WEITSCHIES, Werner, D-10961 Berlin (DE); HELDMANN, Dieter, D-10555 Berlin (DE); HAUFF, Peter, D-12627 Berlin (DE); FRITZSCH, Thomas, D-12169 Berlin (DE); STAHL, Harald, D-12205 Berlin (DE)
(86) Internationale Anmeldenummer: EP9402806
(87) Internationale Veröffentlichungsnummer: WO9507072

(56) Entgegenhaltungen:
- EP-A- 0 327 490
- EP-A- 0 504 881
- WO-A-92/19272
- WO-A-92/22298
- WO-A-93/00933
- US-A- 5 190 766
- MACROMOLECULES, Bd.25, 1992 Seiten 123 - 128 LIU L.-S. ET AL 'EXPERIMENTAL APPROACH TO ELUCIDATE THE MECHANISM OF ULTRASOUND-ENHANCED POLYMER EROSION AND RELEASE OF INCORPORATED SUBSTANCES'

## Beschreibung

Die Erfindung betrifft den in den Patentansprüchen gekennzeichneten Gegenstand, das heißt neue wirkstoffhaltige Mikropartikel, die zusätzlich zum (zu den) Wirkstoff(en) mindestens ein Gas bzw. eine gasförmige Phase enthalten, diese Partikel enthaltende Mittel (mikropartikuläre Systeme), deren Verwendung zur ultraschallgesteuerten *in vivo* Wirkstoff-Freisetzung, zur ultraschallunterstützten Zellinkorporation von Wirkstoffen (Sonoporation) sowie Verfahren zur Herstellung der Partikel und Mittel.

Mikropartikuläre Systeme zur kontrollierten Wirkstofffreigabe gibt es schon seit vielen Jahren. Eine Vielzahl an möglichen Hüllsubstanzen und Wirkstoffen läßt sich hierzu verwenden. Ebenso gibt es eine ganze Reihe unterschiedlicher Herstellungsverfahren. Zusammenstellungen über die verwendeten Hüllsubstanzen und Herstellungsverfahren finden sich z.B. bei: M. Bornschein, P. Melegari, C. Bismarck, S. Keipert: Mikro- und Nanopartikeln als Arzneistoffträgersysteme unter besonderer Berücksichtigung der Herstellungsmethoden, Pharmazie 44 (1989) 585-593 und M. Chasin, R. Langer (eds.): Biodegradable Polymers as Drug Delivery Systems, Marcel Dekker, New York, 1990.

Die Freisetzung von Wirkstoffen aus mikropartikulären Systemen beruht überwiegend auf Diffusions- oder Erosionsprozessen [vgl. C. Washington: Drug release from microdisperse systems: A critical review, Int. J. Pharm. 58 (1990) 1-12 und J. Heller: Bioerodible Systems, *in:* R.S. Langer, D.L. Wice (eds.): Medical applications of controlled release Vol. 1, CRC Press, Florida, 1984, p. 69-101].

Diese Prinzipien sind jedoch mit dem Nachteil behaftet, daß die zeitliche Steuerbarkeit der Wirkstofffreisetzung aus mikrodispersen Systemen *in vivo* auf die Geschwindigkeit des Erosionsprozesses und/oder Diffusionsprozesses begrenzt ist und nach Applikation nicht weiter beeinflußt werden kann.

Die bislang bekannten Konzepte zur örtlichen Steuerung der Wirkstofffreisetzung *in vivo* aus mikropartikulären Systemen beruhen fast ausschließlich entweder auf unspezifischen Anreicherungen der mikropartikulären Wirkstoffträger in bestimmten Zielorganen wie Leber und Milz oder auf Maßnahmen zur gezielten Veränderung der Organverteilung *in vivo* nach Applikation durch die Veränderung der Oberflächeneigenschaften der mikropartikulären Systeme mit Hilfe von Tensiden oder spezifitätsvermittelnden Stoffen wie z.B. Antikörpern [vgl.: R.H. Müller: Colloidal carriers for controlled drug delivery - Modification, characterization and in vivo distribution -, Kiel, 1989; S. D. Tröster, U. Müller, J. Kreuter: Modification of the biodistribution of poly(methylmethacrylate) nanoparticles in rats by coating with surfactants, Int. J. Pharm. 61(1991), 85-100; S.S. Davis, L. Illum, J.G. Mcvie, E. Tomlinson (eds.): Microspheres and drug therapy, Elsevier science publishers B.V., 1984 und H. Tsuji, S. Osaka, H. Kiwada: Targeting of liposomes surface-modified with glycyrrhizin to the liver, Chem. Pharm. Bull. 39 (1991) 1004-1008]. Alle diese Verfahren bieten darüber hinaus jedoch keine weitere Möglichkeit den Ort der Wirkstofffreisetzung nach Applikation aktiv zu beeinflussen. Des weiteren ist es nicht möglich, das Ausmaß und die Geschwindigkeit der Wirkstofffreigabe nach Applikation zu beeinflussen.

Erste Versuche, aktiv den Ort der Wirkstofffreisetzung zu beeinflussen, beruhen auf der Möglichkeit, vorhandene, bzw. induzierte pH- oder Temperaturdifferenzen zur Freisetzung zu benutzen [vgl.: H. Hazemoto, M. Harada, N. Kamatsubara, M. Haga, Y. Kato: PH-sensitive liposomes composed of phosphatidyl-ethanolamine and fatty acid, Chem. Pharm. Bull. 38 (1990) 748-751 und J.N. Weinstein, R.L. Magin, M.B. Gatwin, D.S. Zaharko: Liposomes and local hyperthermia, Science 204 (1979) 188-191]. Diese Methoden sind jedoch mit dem Nachteil behaftet, daß sie entweder begrenzt sind auf Fälle wo die erforderlichen Temperatur- bzw. pH-Differenzen bereits vorliegen (z.B. im Tumorgewebe) oder die entsprechenden zur Freisetzung erforderlichen Parameter nur durch aufwendige, z.T. invasive Maßnahmen herbeigeführt werden müssen. Darüber hinaus ist im letzteren Fall die örtliche Auflösung gering.

Ein weiteres bekanntes Verfahren, den Ort der Wirkstofffreisetzung zu beeinflussen, besteht in der Verwendung von Mikropartikeln, die durch in den Partikeln verkapselte Ferrofluide über äußerlich angelegte Magnetfelder innerhalb bestimmter Körpersegmente anreicherbar sind [K.J. Widder, A.E. Senyei: Magnetic microspheres: A vehicle for selective targeting of drugs, Pharmac. Ther. 20 (1983) 377-395]. Die Verwendung derartiger Mikropartikel erfordert allerdings die gleichzeitige gezielte Anwendung starker, fokussierbarer Magnetfelder. Magnete, die derartige Felder erzeugen, sind jedoch in der Medizin wenig verbreitet. Desweiteren läßt sich die Geschwindigkeit der Wirkstofffreigabe auf diese Weise nicht beeinflussen.

In der U.S. Patentschrift 4,657,543 wird ein Verfahren beschrieben, bei dem die Freisetzung durch Ultraschalleinwirkung auf wirkstoffhaltige Polymerblöcke hervorgerufen wird. Dieser Effekt beruht im wesentlichen auf einer verstärkten Erosion des Polymers unter Schalleinwirkung. Der Nachteil dieses Verfahrens ist, daß es nur für ortsfeste Implantate geeignet ist. Für deutliche Effekte ist zudem die Verwendung sehr hoher Schalldrücke oder von Dauerschallsignalen notwendig, die zur Gewebeschädigung führen können.

In der WO 92/22298 werden Liposomen beschrieben, die sich durch Einstrahlung von Ultraschall, der im Bereich der Resonanzfrequenz der Mikrobläschen liegt, zerstören lassen. Dabei tritt der verkapselte Wirkstoff aus. Die Resonanzfrequenz wird mit ca. 7,5 MHz angegeben. Diagnostischer Ultraschall derart hoher Frequenz weist jedoch aufgrund der hohen Absorption durch Körpergewebe nur eine geringe Eindringtiefe (wenige Zentimeter) auf. Die beschriebenen Liposomen sind deshalb nur für die Freisetzung von Wirkstoffen in oberflächennahen Gebieten des Körpers geeignet.

Bei der Verwendung von Nukleinsäuren als Wirkstoffe werden in der Literatur zwei Systeme basierend auf viralen Vektoren bzw. nicht-virale Vektoren beschrieben. In vivo werden derzeit als virale Vektoren Retro-, Adeno- und Herpesviren (bzw. deren Rekombinanten) und als nicht-virale Vektoren Liposomen und Liganden zelloberflächenspezifischer Rezeptoren untersucht (G.Y. Wu & C. H. Wu: Delivery systems for gene therapy, Biotherapy, 3 (1991) 87-95 und F. D. Ledley: Are contemporary methods for somatic gene therapy suitable for clinical applications?, Clin Invest Med 16 (1) (1993) 78-88.
Erste Untersuchungen zur Verwendung der Gentherapie beim Menschen konzentrieren sich auf genetisch bedingte Erkrankungen wie z. B. alpha-1-Antitrypsinmangel, zystische Fibrose, Adenosindesaminasemangel und maligner Tumoren wie z. B. Melanome, Mammatumore und Intestinalkarzinome. Bislang sind jedoch keine Vektoren bekannt, die sowohl eine räumliche als auch zeitliche Steuerung der Freisetzung von Nukleinsäuren ermöglichen.

In der U.S. Patentschrift 5,190,766 werden käufliche Albumin-Mikrokapseln (Albunex®) mit 5-Fluoruracil (5-FU) gemischt. Es wird im Ergebnis eine wäßrige Suspension erhalten, die einen nicht unerheblichen Anteil freien Wirkstoffes im Suspensionsmedium enthält.
Der freie Wirkstoffanteil in derartigen Präparationen verteilt sich aber nach Applikation naturgemäß von außen nicht steuerbar im Blut. Dies führt zu einer Reihe von unerwünschten Effekten. Sensible Wirkstoffe (wie z.B. Nukleinsäuren) können auf dem Weg zum Wirkort bereits durch Nukleasen gespalten werden. Außerdem kann der freie Wirkstoff unerwünschte Nebenwirkungen an anderen Organen hervorrufen. Dies ist vor allem bei Zytostatika wie 5-FU zu erwarten. Desweiteren wird der Körper wie bei herkömmlichen Präparationen mit der Metabolisierung "überflüssigen" Wirkstoffes belastet.

Es besteht daher für vielfältige Zwecke weiterhin ein Bedarf an gezielt applizierbaren Formulierungen, die die genannten Nachteile des Standes der Technik überwinden, d.h. bei denen sowohl der Ort und Zeitpunkt der Wirkstofffreisetzung als auch die Menge der abgegebenen Substanz, gezielt durch einfache, nicht invasive Maßnahmen gesteuert werden kann. Die Formulierungen sollten darüber hinaus den Wirkstoff ausschließlich im Kern enthalten und eine hohe Stabilität, insbesondere in Hinblick auf mechanische Einflüsse, aufweisen.

Der Erfindung liegt somit die Aufgabe zugrunde, derartige Formulierungen zur Verfügung zu stellen, sowie Verfahren zu ihrer Herstellung zu schiffen.

Diese Aufgabe wird durch die Erfindung gelöst.

Es wurde gefunden, daß bei mikropartikulären Systemen, die zusammengesetzt sind aus einem pharmazeutisch verträglichen Suspensionsmedium und Mikropartikeln, die aus einer bioabbaubaren Hülle und einem gas- und wirkstoffhaltigen Kern bestehen, überraschenderweise bei der Bestrahlung mit diagnostischen Ultraschallwellen in einem Frequenzbereich der unterhalb der Resonanzfrequenz der Partikel liegt, die Hülle dieser Partikel zerstört wird und so der (die) verkapselte(n) Wirkstoff(e) gezielt freigesetzt wird (werden).

Die Erfindung betrifft somit neue wirkstoffhaltige Mikropartikel, die neben dem Wirkstoff ein Gas, eine gasförmige Phase oder Gasgemische enthalten, sowie mikropartikuläre Systeme bestehend aus den erfindungsgemäßen Mikropartikeln sowie einem pharmazeutisch verträglichen Suspensionsmedium.

Die Partikel weisen eine Dichte Meiner als 0,8 g/cm³, bevorzugt kleiner als 0,6 g/cm³ auf und haben eine Größe im Bereich von 0,1 - 8 µm, vorzugsweise 0,3 - 7 µm. Im Falle von verkapselten Zellen beträgt die bevorzugte Partikelgröße 5-10 µm. Aufgrund der geringen Größe verteilen sie sich nach i.v. Injektion innerhalb des gesamten Gefäßsystems. Unter Sichtkontrolle auf dem Monitor eines diagnostischen Ultraschallgerätes kann dann durch Intensivierung des Schallsignals eine vom Anwender gesteuerte Freisetzung der enthaltenen Stoffe herbeigeführt werden, wobei die zur Freisetzung erforderliche Frequenz unterhalb der Resonanzfrequenz der Mikropartikel liegt. Geeignete Frequenzen liegen im Bereich von 1 - 6 MHz, bevorzugt zwischen 1,5 und 5 MHz.

Dadurch ist erstmalig innerhalb des gesamten Körpers eine kombinierte Steuerung der Wirkstofffreigaberate und des Wirkstofffreigabeortes durch den Anwender möglich.

Diese Freisetzung, durch Zerstörung der Partikelhülle, ist überraschenderweise auch mit Ultraschallfrequenzen weit unterhalb der Resonanzfrequenz der Mikrobläschen mit in der medizinischen Diagnostik üblichen Schalldrücken möglich, ohne daß es zu einer Erwärmung des Gewebes kommt. Dieses ist besonderes deswegen bemerkenswert, weil auf Grund der großen mechanischen Stabilität der Partikelhülle - wie sie z.B. in Hinblick auf Lagerstabilität von Vorteil ist - eine Zerstörung der Hülle mit relativ energiearmer Strahlung nicht zu erwarten war.

Die Wirkstofffreigabe kann aufgrund des hohen Gasanteils der Partikel und der damit verbundenen Echogenität, *in vivo* über die Abnahme des empfangenen Ultraschallsignals kontrolliert werden.

Weiterhin wurde gefunden, daß bei Anwendung erfindungsgemäßer mikropartikulärer Systeme ein verbesserter Transfer von Wirkstoffen in die Zellen erzielt werden kann (Sonoporation).

Außerdem wurde gefunden, daß die aus den erfindungsgemäßen mikropartikulären Systemen freigesetzten Wirkstoffe im Vergleich zu dem reinen Wirkstoff überraschenderweise eine erhöhte pharmakologische Wirksamkeit zeigen.

Die erfindungsgemäßen mikropartikulären Systeme sind aufgrund ihrer Eigenschaften für eine gezielte Freisetzung von Wirkstoffen und deren erhöhten Transfer in die Zielzellen unter Einwirkung von diagnostischem Ultraschall geeignet.

Als Hüllmaterialien für die Gas/Wirkstoff enthaltenden Mikropartikel eignen sich prinzipiell alle biologisch abbaubaren und physiologisch verträglichen Materialien, wie z.B. Proteine wie Albumin, Gelatine, Fibrinogen, Collagen sowie deren Derivate wie z.B. succinylierte Gelatine, quervenetzte Polypeptide, Umsetzungsprodukte von Proteinen mit Polyethylenglykol (z.B. mit Polyethylenglykol konjugiertes Albumin), Stärke oder Stärkederivate, Chitin, Chitosan, Pektin, biologisch abbaubare synthetische Polymere wie Polymilchsäure, Copolymere aus Milchsäure und Glykolsäure, Polycyanoacrylate, Polyester, Polyamide, Polycarbonate, Polyphosphazene, Polyaminosäuren, Poly-ε-caprolacton sowie Copolymere aus Milchsäure und ε-Caprolacton und deren Gemische. Besonders geeignet sind Albumin, Polymilchsäure, Copolymere aus Milchsäure und Glykolsäure, Polycyanoacrylate, Polyester, Polycarbonate, Polyaminosäuren, Poly-ε-caprolacton sowie Copolymere aus Milchsäure und ε-Caprolacton.

Das (die) eingeschlossene(n) Gas(e) können beliebig gewählt werden, wobei jedoch physiologisch unbedenkliche Gase wie Luft, Stickstoff, Sauerstoff, Edelgase, halogenierte Kohlenwasserstoffe, SF₆ oder deren Gemische bevorzugt sind. Ebenfalls geeignet sind Ammoniak, Kohlendioxid sowie dampfförmige Flüssigkeiten, wie z.B. Wasserdampf oder niedrigsiedende Flüssigkeiten (Siedepunkt < 37 °C).

Der pharmazeutische Wirkstoff kann ebenfalls beliebig gewählt werden. Als Beispiele seien genannt Arzneistoffe, Toxine, Viren, Virusbestandteile, Bestandteile von bakteriologischen Zellwänden, Peptide wie z.B.Endothelin, Proteine, Glycoproteine, Hormone, lösliche Botenstoffe, Farbstoffe, Komplement Komponenten, Adjuvantien, trombolytische Agentien, tumornekrose Faktoren, Zytokine (wie z.B. Interleukine, koloniestimulierende Faktoren wie GM-CSF, M-CSF, G-CSF) und/oder Prostaglandine. Die erfindungsgemäßen Mikropartikeln eignen sich insbesondere zur Verkapselung von Nukleinsäuren, ganzen Zellen und/oder Zellbestandteilen, die (z.B. bei der Gentherapie) im Zielorgan mittels Ultraschall freigesetzt werden sollen.

Der Begriff pharmazeutischer Wirkstoff schließt sowohl die natürlichen, als auch die synthetisch oder gentechnologisch hergestellten Wirkstoffe ein.

Bevorzugt werden pharmazeutische Wirkstoffe verwendet, deren applizierte Dosis (bei bolusförmiger Injektion) 100 mg pro Anwendung nicht übersteigt. Dabei ist zu berücksichtigen, daß bei den erfindungsgemäßen mikropartikulären Systemen, wie zuvor beschrieben, eine Erhöhung der pharmakologischen Wirksamkeit erreicht wird, wobei in verschiedenen Fällen eine Wirkungsverstärkung beobachtet werden kann, wodurch die erfindungsgemäßen mikropartikulären Systeme auch für Wirkstoffe einsetzbar sind, die auf konventionellem Wege im Bolus höher als 100 mg pro Anwendung dosiert werden müssen.

Sind noch höhere Dosierungen erforderlich, so empfiehlt es sich die Mittel über einen längeren Zeitraum als Infusionslösung zu verabreichen

Obgleich es über die genannten Limitierungen hinaus keine weiteren Einschränkungen gibt, können die erfindungsgemäßen mikropartikulären Systeme besonders dort mit Vorteil eingesetzt werden, wo es aufgrund einer geringen *in vivo* Lebensdauer des Wirkstoffs in freier Form nicht oder nur in beschränktem Ausmaß möglich ist, das Zielorgan zu erreichen, ohne daß zuvor Zersetzung des Wirkstoffs eingetreten ist. Zu derartigen Wirkstoffen zählen verschiedene Hormone, Peptide, Proteine, Zellen und deren Bestandteile sowie Nukleinsäuren.

Ein Verfahren zur Herstellung der erfindungsgemäßen Mikropartikel besteht darin, daß zunächst in an sich bekannter Weise (DE 38 03 972, WO 93/00933, EP 0 514 790, WO 92/17213, US 5,147,631, WO 91/12823, EP 0 048 745) gasgefüllte Mikropartikel hergestellt werden. Erfindungsgemäß werden diese dann mit in überkritischen Gasen gelösten Wirkstoffen befüllt. Dazu werden die mit geeigneten Verfahren getrockneten (z.B. Gefriertrocknung) gashaltigen Mikropartikel mit einer Lösung des Wirkstoffs in einem überkritischen Gas in einem Autoklaven behandelt. Zweckmäßigerweise verfährt man, indem Wirkstoff und gasgefüllter Mikropartikel gemeinsam in einem Autoklaven vorgelegt werden und dieser anschließend mit dem überkritischen Gas oder Gasgemisch befüllt wird. Als überkritische Gase eignen sich je nach Wirkstoff alle Gase, die in einen überkritischen Zustand überführt werden können, insbesondere jedoch überkritisches Kohlendioxid, überkritischer Stickstoff, überkritischer Ammoniak sowie überkritsche Edelgase. Nach der Behandlung der Mikropartikel mit der Lösung des Wirkstoffs im überkritischen Gas oder Gasgemisch wird der überschüssige Wirkstoff an der äußeren Oberfläche der Mikropartikel falls erforderlich durch Waschen der Mikropartikel in einem geeigneten Medium entfernt und die so gereinigten Partikel gewünschtenfalls gefriergetrocknet. Dieses Verfahren ist für alle Wirkstoffe geeignet, die sich in überkritischen Gasen oder Gasgemischen lösen, wie z.B. Peptide oder lipophile Arzneistoffe.

Ein alternatives Verfahren, das sich insbesondere zur Verkapselung von Wirkstoffen die in überkritischen Gasen oder Gasgemischen unlöslich sind (wie z.B. Proteine, zuckerhaltige Verbindungen), eignet, beruht auf der Verkapselung einer wirkstoffhaltigen wäßrigen Phase mittels einer Mehrfachemulsion. Als besonders geeignet haben sich Wasser/Öl/Wasser (W/O/W)-Emulsionen erwiesen. Dazu wird das Hüllmaterial in einem geeigneten organischen Lösungsmittel, das nicht in Wasser löslich ist, in einer Konzentration von 0,01 - 20 % (m/V) gelöst. In diese Lösung wird eine wäßrige Lösung des zu verkapselnden Wirkstoffs so emulgiert, daß eine Emulsion vom Typ W/O entsteht. Beide Lösungen können zusätzlich Hilfsstoffe wie Emulgatoren enthalten. Bevorzugt ist es jedoch, aus Gründen der im allgemeinen begrenzten biologischen Verträglichkeit von Emulgatoren, auf diese weitgehend zu verzichten. Als vorteilhaft hat es sich erwiesen, der inneren wäßrigen Phase pharmazeutisch akzeptable Quasiemulgatoren wie z.B. Polyvinylalkohol, Polyvinylpyrrolidon, Gelatine, Albumin oder Dextrane im Konzentrationsbereich von 0,1 bis 25 % zuzusetzen. Als besonders vorteilhaft hat es sich erwiesen, in der inneren wäßrigen Phase, gegebenenfalls zusätzlich zu den anderen verwendeten Hilfsstoffen, 0,1 - 20 % (m/V) eines gut wasserlöslichen pharmazeutisch akzeptablen Salzes oder Zuckers oder Zuckeralkohols, wie z.B. Natriumchlorid, Galaktose, Mannitol, Laktose, Saccharose, Glukose, Natriumhydrogenphosphat zu lösen. Es kann außerdem vorteilhaft sein, die innere wäßrige Phase vor der Emulgierung mit der verwendeten organischen Phase zu sättigen. Die hergestellte Emulsion vom Typ W/O sollte eine mittlere Tröpfchengröße der inneren Phase von ca. 0,1 bis 10 µm aufweisen. Diese Emulsion wird unter Rühren in das mindestens gleiche Volumen einer wäßrigen Lösung eines Emulgators oder Quasiemulgators gegeben. Das organische Lösungsmittel wird unter Rühren durch geeignete Verfahren (solvent evaporation) wieder entfernt. Die erhaltenen wassergefüllten Mikropartikel werden erforderlichenfalls gewaschen und anschließend so getrocknet, daß die innere Wasserphase ohne Zerstörung der Mikropatikel entfernt wird. Grundsätzlich geeignete Trocknungsverfahren sind die Gefriertrocknung und die Sprühtrocknung. Bevorzugt ist die Gefriertrocknung. Dazu wird in der Suspension der Mikropartikel ein gerüstbildender Hilfsstoff wie z.B. Zucker, Zuckeralkohole, Gelatine, Gelatine-Derivate, Albumin, Aminosäuren, Polyvinylpyrrolidon, Polyvinylalkohol in einer Konzentration von ca. 0,5 - 20 % (m/V) gelöst. Die Suspension wird anschließend bei möglichst tiefen Temperaturen, bevorzugt unterhalb ca. -30 °C eingefroren und dann gefriergetrocknet. Nach der Gefriertocknung und Redispergierung in einem geeigneten Suspensionsmedium, lassen sich die entstandenen gashaltigen Mikropartikel der erforderlichen Dichte durch Flotation oder Zentrifugation, von eventuell ebenfalls vorhandenen soliden oder immer noch wassergefüllten Mikropartikeln abtrennen und falls erforderlich, möglichst unter Zusatz von Gerüstbildnern, erneut gefriertrocknen. Die Mikropartikel enthalten dann den verkapselten Wirkstoff und Gas bzw. gasförmige Phase nebeneinander.

Die Herstellung der erfindungsgemäßen mikropartikulären Systeme aus den nach den vorbeschriebenen Verfahren hergestellten Partikel erfolgt durch Resuspendieren der Partikel in einem pharmazeutisch verträglichen Suspensionsmedium. Das Resuspendieren in einem geeigneten Medium kann sich unmittelbar an den letzten Verfahrensschritt (die Gefriertrocknung) anschließen, kann aber gewünschtenfalls auch erst durch den behandelnden Arzt vor der Anwendung erfolgen. In letzterem Fall liegen die erfindungsgemäßen mikropartikulären Systeme als ein Kit, bestehend aus einem ersten die Partikel enthaltenden Behälter und einem zweiten das Suspensionsmedium enthaltenden Behälter, vor. Die Größe des ersten Behälters ist so zu wählen, daß auch das Suspensionsmedium in diesem vollständig Platz findet. So kann z.B. mittels einer Spritze über eine im Verschluß des ersten Behälters befindliche Membran, das Suspensionsmedium vollständig zu den Partikeln gegeben werden und durch anschließendes Schütteln die injektionsfertige Suspension hergestellt werden. Als Suspensionsmedien kommen alle dem Fachmann bekannten injizierbaren Medien infrage, wie z.B. physiologische Kochsalzlösung, Wasser p.i. oder 5 %ige Glukoselösung.

Die applizierte Menge richtet sich nach dem jeweilig eingeschlossenen Wirkstoff. Als orientierender oberer Grenzwert kann ein Wert angenommen werden, wie er auch bei konventioneller Verabreichung des jeweiligen Wirkstoffs verwendet werden würde. Aufgund des wirkungsverstärkenden Effekts sowie der Möglichkeit den Wirkstoff spezifisch aus den erfindungsgemäßen mikropartikulären System freizusetzen, liegt die erforderliche Dosis im allgemeinen jedoch unter diesem oberen Grenzwert.

Die nachfolgenden Beispiele dienen der Erläuterung des Erfindungsgegenstandes, ohne ihn auf diese beschränken zu wollen.

### Beispiel 1: Coffein-haltige Mikropartikel aus Polycyanacrylat

Gasgefüllte Mikropartikel, die aus Butylcyanacrylsäure gemäß DE 38 03 972 hergestellt wurden, werden unter Zusatz von 2 % (m/V) Polyvinylalkohol gefriergetrocknet. Es werden ca. 3 · 10⁹ Partikel in Form des Lyophilisats zusammen mit 50 mg Coffein in einen Autoklaven gefüllt. Das Gemisch wird bei ca. 45 °C und 100-120 bar mit Kohlendioxid behandelt. Die Entfernung des überschüssigen Coffeins wird folgendermaßen durchgeführt: Die dem Autoklaven entnommenen Mikropartikel werden in 3 ml Wasser, das 1 % Lutrol F 127 gelöst enthält, resuspendiert. Die Partikel werden durch Zentrifugation abgetrennt und in 3 ml Wasser, das 1 % Lutrol F 127 gelöst enthält, resuspendiert. Die Zentrifugation mit anschließender Redispergierung in 3 ml Wasser, das 1 % Lutrol F 127 gelöst enthält, wird solange wiederholt, bis im Wasser kein Coffein mehr photometrisch bei 273 nm nachgewiesen werden kann.

### Beispiel 2: Fibrinolytische Mikropartikel aus Poly (D,L-Milchsäure-Glykolsäure)

2 g Poly (D,L-Milchsäure-Glykolsäure) (50:50) (Resomer RG 503, Boehringer Ingelheim) werden in 20 ml CH₂Cl₂ gelöst. 10 mg r t-PA (Gewebsplasminogenaktivator) werden in 4 ml einer 4 %igen wässrigen Gelatinelösung, die zuvor autoklaviert wurde, gelöst und unter Rühren mit einem schnellaufenden Rührwerk zu der organischen Phase gegeben. Nach vollständiger Emulgierung werden 200 ml einer 4 %igen autoklavierten Gelatinelösung unter weiterem Rühren zugegeben. Die Emulsion wird 8 h bei Raumtemperatur gerührt. Die entstandenen Partikel werden durch einen 5 µm-Filter filtriert, durch Zentrifugation separiert, in 50 ml 4 %iger autoklavierter Gelatinelösung resuspendiert, bei -78 °C eingefroren und gefriergetrocknet. Nach Resuspendierung werden die gashaltigen Mikropartikel durch Zentrifugation (bei 1000 Upm, 30 min) abgetrennt. Die gashaltigen Mikropartikel werden in 20 ml Wasser für Injektionszwecke aufgenommen. Sie weisen eine Dichte kleiner als 0,7 g/cm³ auf.

### Beispiel 3: in vitro Freisetzung von Coffein durch Ultraschall

1 ml einer nach Beispiel 1 zubereiteten Partikelsuspension, mit Wasser verdünnt auf eine Konzentration von 10⁸ Partikel/ml wird in ein mit 100 ml entgastem Wasser gefülltes Becherglas gegeben. In das Wasser wird ein 3,5 MHz Schallkopf eines diagnostischen Ultraschallgerätes (HP Sonos 1000) getaucht und die Veränderung des B-Bildes beobachtet. Zunächst wird das Gerät mit einer mittleren Schalleistung (Transmit ≤ 20 dB) betrieben, wobei deutliche Echos zu erkennen sind. Eine Prüfung des partikelfreien Wassers auf Coffein bleibt negativ. Wird der Schalldruck erhöht (Transmit > 30 dB), verschwinden die Echos. Die Flüssigkeit enthält nun nachweisbares freies Coffein, mikroskopisch sind überwiegend Bruchstücke der Mikropartikel zu erkennen und nur noch sehr wenige intakte.

### Beispiel 4: in vitro Freisetzung von rekombinantem tissue Plasminogen Aktivator (r t-PA) durch Ultraschall

1 ml einer nach Beispiel 2 zubereiteten Partikelsuspension, mit Wasser verdünnt auf eine Konzentration von 108 Partikel/ml wird in ein mit 100 ml entgastem Wasser gefülltes Becherglas gegeben. In das Wasser wird ein 3,5 MHz Schallkopf eines diagnostischen Ultraschallgerätes (HP Sonos 1000) getaucht und die Veränderung des B-Bildes beobachtet. Zunächst wird das Gerät mit einer geringen Schalleistung (Transmit ^{∼} 10 dB) betrieben, wobei deutliche Echos zu erkennen sind. Eine Prüfung des partikelfreien Wassers auf r t-PA bleibt negativ. Wird der Schalldruck erhöht (Transmit > 30 dB), verschwinden die Echos. Die Flüssigkeit enthält nun nachweisbares freies r t-PA, mikroskopisch sind überwiegend Bruchstücke der Mikropartikel zu erkennen und nur noch sehr wenige intakte. Die mit dem erhöhten Schalldruck behandelte Partikelsuspension weist fibrinolytische Eigenschaften auf

### Beispiel 5: Mitomycin-haltige Mikropartikel aus Polymilchsäure

2 g Polymilchsäure (MG ca. 20 000) werden in 100 ml CH₂Cl₂ gelöst. 20 mg Mitomycin werden in 15 ml 0,9 %iger wässriger Kochsalzlösung gelöst und unter Rühren mit einem schnellaufenden Rührwerk zu der organischen Phase gegeben. Nach vollständiger Emulgierung werden 200 ml einer 1 %igen Lösung von Polyvinylalkohol (MG ca. 15 000) in Wasser unter weiterem Rühren zugegeben. Die Emulsion wird 4 h bei Raumtemperatur gerührt. Die entstandenen Partikel werden durch einen 5 µm-Filter filtriert, durch Zentrifugation separiert, in 50 ml einer 5 %igen Lösung von Polyvinylpyrrolidon (MG ca. 10 000) in Wasser resuspendiert, bei -50 °C eingefroren und anschließend gefriergetrocknet. Nach Resuspendierung werden die gashaltigen Mikropartikel durch Zentrifugation (bei 1000 Upm, 30 min) abgetrennt. Die gashaltigen Mikropartikel werden in 20 ml Wasser für Injektionszwecke aufgenommen. Sie weisen eine Dichte Meiner als 0,7 g/cm³ auf. Sie eignen sich auch als Kontrastmittel für Ultraschall und setzen bei Beschallung mit diagnostischem Ultraschall Mitomycin frei.

### Beispiel 6: Vincristinsulfat-haltige Mikropartikel aus Poly-ε-caprolacton

2 g Poly-ε-caprolacton (MG ca. 40 000) werden in 50 ml CH₂Cl₂ gelöst. 10 mg Vincristinsulfat werden in 15 ml einer 5 %igen wässrigen Lösung von Galactose gelöst und unter Rühren mit einem schnellaufenden Rührwerk zu der organischen Phase gegeben. Nach vollständiger Emulgierung werden 200 ml einer 5 %igen Lösung von Humanalbumin in Wasser unter weiterem Rühren zugegeben. Die Emulsion wird 4 h bei Raumtemperatur gerührt. Die entstandenen Partikel werden durch einen 5 µm-Filter filtriert, durch Zentrifugation separiert, in 50 ml einer 5 %igen Lösung von Humanalbumin in Wasser resuspendiert, bei -50 °C eingefroren und anschließend gefriergetrocknet. Nach Resuspendierung werden die gashaltigen Mikropartikel durch Zentrifugation (bei 1000 Upm, 30 min) abgetrennt. Die gashaltigen Mikropartikel weisen eine Dichte Meiner als 0,7 g/cm³ auf. Sie eignen sich als Kontrastmittel für Ultraschall und setzen bei Beschallung mit diagnostischem Ultraschall Vincristinsulfat frei.

### Beisiel 7: Ilomedin-haltige Mikropartikel aus Polycyanacrylsäurebutylester

3 g Polycyanacrylsäurebutylester werden in 50 ml CH₂Cl₂ gelöst. 1 mg Ilomedin wird in 15 ml einer 5 %igen wässrigen Lösung von Galactose gelöst und unter Rühren mit einem schnellaufenden Rührwerk zu der organischen Phase gegeben. Nach vollständiger Emulgierung werden 200 ml einer 2,5 %igen Lösung von Polyvinylalkohol (MG 15 000) in Wasser unter weiterem Rühren zugegeben. Die Emulsion wird 4 h bei Raumtemperatur gerührt. Die entstandenen Partikel werden durch einen 5 µm-Filter filtriert, durch Zentrifugation separiert, in 50 ml einer 10 %igen Lösung von Lactose in Wasser resuspendiert, bei -50 °C eingefroren und anschließend gefriergetrocknet. Nach Resuspendierung werden die gashaltigen Mikropartikel durch Zentrifugation (bei 1000 Upm, 30 min) abgetrennt. Die gashaltigen Mikropartikel weisen eine Dichte kleiner als 0,7 g/cm³ auf. Sie eignen sich als Kontrastmittel für Ultraschall und setzen bei Beschallung mit diagnostischem Ultraschall Ilomedin frei.

### Beispiel 8: Methylenblau-haltige Mikropartikel aus Poly(D,L-Milchsäure-Glykolsäure)

4 g Poly (D,L-Milchsäure-Glykolsäure) (50:50) (Resomer RG 503, Boehringer Ingelheim) werden in 50 ml CH₂Cl₂ gelöst. 20 mg Methylenblau werden in 4 ml einer 4 %igen wässrigen Gelatinelösung, die zuvor autoklaviert wurde, gelöst und unter Rühren mit einem schnellaufenden Rührwerk zu der organischen Phase gegeben. Nach vollständiger Emulgierung werden 200 ml einer 4 %igen autoklavierten Gelatinelösung unter weiterem Rühren zugegeben. Die Emulsion wird 8 h bei Raumtemperatur gerührt. Die entstandenen Partikel werden durch einen 5 µm-Filter filtriert, durch Zentrifugation separiert, in 50 ml 4 %iger autoklavierter Gelatinelösung resuspendiert, bei -78 °C eingefroren und gefriergetrocknet. Nach Resuspendierung werden die gashaltigen Mikropartikel durch Zentrifugation (bei 1000 Upm, 30 min) abgetrennt. Die gashaltigen Mikropartikel werden in 20 ml Wasser für Injektionszwecke aufgenommen. Sie weisen eine Dichte kleiner als 0,7 g/cm³ auf und setzen bei Beschallung mit Ultraschall (Schalldruck >50dB, Frequenz 2,5MHz) Methylenblau frei.

### Beispiel 9: Nukleinsäurehaltige Mikropartikel (Markergen β-Gal + Albuminpromotor) aus Poly(D,L-Milchsäure-Glykolsäure)

0,4 g Polyvinylpyrrolidon (k < 18) und 2 g eines Copolymeren aus Milchsäure und Glykolsäure 50:50 werden in 50 ml CH₂Cl₂ gelöst. Unter Rühren werden 5 ml einer Lösung von 300 µg Markergen β-Gal mit Albuminpromotor in 0,9 %iger Kochsalzlösung zugegeben. Die entstandene Emulsion wird unter Rühren in 200 ml einer 2 %igen autoklavierten (121 °C, 20 min) Gelatinelösung überführt. Nach 3 h wird die entstandene Suspension in Portionen à 5 ml abgefüllt, bei -55 °C eingefroren und anschließend 70 h gefriergetrocknet. Die Vials werden nach Gefriertrocknung mit je 5 ml Wasser resuspendiert und 3 h stehen gelassen. Die flotierten Partikel werden abgenommen in je 2 ml Wasser, das 10 % PVP enthält, resuspendiert und nach Einfrieren bei -55 °C erneut 90 h gefriergetrocknet.

### Beispiel 10: In-vitro-Freisetzung von Nukleinsäure (Markergen β-Gal + Albuminpromotor) aus Mikropartikeln

1 Vial einer nukleinsäurehaltigen Mikropartikelpräparation, hergestellt nach Beispiel 9, wird mit 2 ml Wasser resuspendiert. 1 ml der Suspension wird mit Ultraschall behandelt (Probe 1), 1 ml wird nicht mit Ultraschall behandelt (Probe 2). Beide Proben werden zentrifugiert, die partikelarmen Phasen entnommen, durch einen 0,2 µm Filter filtriert. Die Filtrate werden mittels Gelelektrophorese auf ihren Gehalt an Markergen β-Gal untersucht. Das Filtrat aus Probe 1 enthält ca. 100 % mehr β-Gal als das Filtrat aus Probe 2.

### Beispiel 11: In-vivo-Freisetzung von Nukleinsäure (Markergen β-Gal + Albuminpromotor) aus Mikropartikeln

2 Vials einer nukleinsäurehaltigen Mikropartikelpräparation, hergestellt nach Beispiel 9 werden mit je 2 ml Wasser resuspendiert. Das gesamte Resuspendat wird der narkotisierten Ratte langsam i. v. (ca. 0,5 ml/min) appliziert. Während der Applikation erfolgt eine Beschallung (7,5 MHz) der Leber, welche bis zu 20 min nach Injektionsabschluß fortgeführt wird. 48 Stunden nach der Partikelgabe wird die Leber entnommen und bei -40 °C in Isopentan schockgefroren. Der enzymhistochemische Nachweis der neutralen β-Galaktosidase erfolgt an 8 - 10 µm dicken Gefrierschnitten. Die Gegenfärbung wird mit Kernechtrot durchgeführt. Im Lebergefrierschnitt stellte sich die neutrale β-Galaktosidase - als Ergebnis der Genexpression - diffus verteilt als dunkelblaue Signale dar.

## Patentansprüche

1. Verwendung von mikropartikulären Systemen, zusammengesetzt aus einem pharmazeutisch verträglichem wirkstofffreiem Suspensionsmedium und Mikropartikeln, bestehend aus einer biologisch abbaubaren Polymerhülle und einem gas- und wirkstoffhaltigen Kern zur Herstellung eines Arzneimittels zur in-vivo Freisetzung des eingeschlossenen Wirkstoffs durch Ultraschall.

2. Verwendung von Mikropartikeln nach Anspruch 1, dadurch gekennzeichnet, daß die Dichte der Partikel kleiner als 0,8 g/cm³ ist.

3. Verwendung von Mikropartikeln nach Anspruch 1, dadurch gekennzeichnet, daß die Partikelgröße 0,1 - 10 µm ist.

4. Verwendung von Mikropartikeln nach Anspruch 1, dadurch gekennzeichnet, daß als biologisch abbaubares Polymer Proteine, Gelatine, Fibrinogen, Collagen sowie deren Derivate, quervenetzte Polypeptide, Umsetzungsprodukte von Proteinen mit Polyethylenglykol, Stärke oder Stärkederivate, Chitin, Chitosan, Pektin, Polymilchsäure, Copolymere aus Milchsäure und Glykolsäure, Polycyanoacrylate, Polyester, Polyamide, Polycarbonate, Polyphosphazene, Polyaminosäuren, Poly-ε-caprolacton sowie Copolymere aus Milchsäure und ε-Caprolacton oder deren Gemische verwendet wird.

5. Verwendung von Mikropartikeln nach Anspruch 1, dadurch gekennzeichnet, daß als Wirkstoff Toxine, Viren, Virusbestandteile, Bestandteile von bakteriologischen Zellwänden, lösliche Botenstoffe, Farbstoffe, Komplement Komponenten, Adjuvantien, trombolytische Agentien, Tumornekrose Faktoren, Nukleinsäuren, Peptide, Proteine, Glykoproteine, Hormone, Zytokine und/oder Prostaglandine enthalten sind.

6. Verwendung von Mikropartikeln nach Anspruch 1, dadurch gekennzeichnet, daß als Wirkstoff Zellen und/oder deren Bestandteile enthalten sind.

7. Verwendung von Mikropartikeln nach Anspruch 1, dadurch gekennzeichnet, daß als gasförmige Phase Luft, Stickstoff, Sauerstoff, Kohlendioxid, Edelgase, Ammoniak, halogenierte oder teilhalogenierte Kohlenwasserstoffe, SF₆ und/oder Wasserdampf oder niedrigsiedende Flüssigkeiten enthalten sind.

8. Verwendung von Mikropartikeln nach Anspruch 1, dadurch gekennzeichnet, daß die in-vivo Freisetzung durch Einstrahlung von diagnostischem Ultraschall erfolgt.

9. Verwendung von Mikropartikeln nach Anspruch 1, dadurch gekennzeichnet, daß die in-vivo Freisetzung durch Einstrahlung von diagnostischem Ultraschall einer Frequenz von 1-6, bevorzugt 1,5 - 5 MHz, erfolgt.

10. Verfahren zur Herstellung von wirkstoffhaltigen Mikropartiklen, dadurch gekennzeichnet, daß gashaltige Mikropartikel mit einer Lösung des Wirkstoffs in einem überkritischen Gas, bevorzugt in überkritischem Kohlendioxid, überkritischem Stickstoff, überkritischem Ammoniak sowie überkritischen Edelgasen, in einem Autoklaven behandelt, anschließend gewaschen und gefriergetrocknet werden.

11. Mikropartikel, enthaltend mindestens einen Wirkstoff und eine Gasphase, hergestellt gemäß Anspruch 10.

12. Mikropartikel, enthaltend eine aus mindestens einem biologisch abbaubaren Polymeren aufgebaute Partikelhülle, eine gasförmige Phase und mindestens eine Nukleinsäure, ein Peptid, ein Protein, ein Glykoprotein, ein Hormon und oder ein Prostaglandin, erhältlich gemäß Anspruch 10.

13. Gefrier- oder sprühgegetrocknete Präparationen, aus denen durch Resuspension mit einem pharmazeutisch verträglichem Medium mikropartikuläre Systeme wie sie gemäß Anspruch 1 verwendet werden, erhalten werden.

## Claims

1. Use of microparticulate systems composed of a pharmaceutically suitable active ingredient-free suspending medium and microparticles consisting of a biodegradable polymer shell and a gas- and active ingredient-containing core for producing a medicinal product for the in vivo release of the enclosed active ingredient by ultrasound.

2. Use of microparticles according to Claim 1, characterized in that the density of the particles is less than 0.8 g/cm³.

3. Use of microparticles according to Claim 1, characterized in that the particle size is 0.1-10 µm.

4. Use of microparticles according to Claim 1, characterized in that proteins, gelatin, fibrinogen, collagen and derivatives thereof, crosslinked polypeptides, products of the reaction of proteins with polyethylene glycol, starch or starch derivatives, chitin, chitosan, pectin, polylactic acid, copolymers of lactic acid and glycolic acid, polycyanoacrylates, polyesters, polyamides, polycarbonates, polyphosphazenes, polyamino acids, poly-ε-caprolactone, and copolymers of lactic acid and ε-caprolactone or mixtures thereof are used as biodegradable polymer.

5. Use of microparticles according to Claim 1, characterized in that toxins, viruses, virus constituents, constituents of bacteriological cell walls, soluble messengers, dyes, complement components, adjuvants, thrombolytic agents, tumour necrosis factors, nucleic acids, peptides, proteins, glycoproteins, hormones, cytokines and/or prostaglandins are present as active ingredient.

6. Use of microparticles according to Claim 1, characterized in that cells and/or their constituents are present as active ingredient.

7. Use of microparticles according to Claim 1, characterized in that air, nitrogen, oxygen, carbon dioxide, inert gases, ammonia, halogenated or partially halogenated hydrocarbons, SF₆ and/or water vapour or low-boiling liquids are present as gaseous phase.

8. Use of microparticles according to Claim 1, characterized in that the in vivo release takes place by beaming in diagnostic ultrasound.

9. Use of microparticles according to Claim 1, characterized in that the in vivo release takes place by beaming in diagnostic ultrasound with a frequency of 1-6, preferably 1.5-5, MHz.

10. Process for producing active ingredient-containing microparticles, characterized in that gas-containing microparticles are treated with a solution of the active ingredient in a supercritical gas, preferably in supercritical carbon dioxide, super-critical nitrogen, supercritical ammonia and supercritical inert gases, in an autoclave, and then washed and freeze-dried.

11. Microparticles comprising at least one active ingredient and one gas phase produced according to Claim 10.

12. Microparticles comprising a particle shell composed of at least one biodegradable polymer, a gaseous phase and at least one nucleic acid, one peptide, one protein, one glycoprotein, one hormone and/or one prostaglandin, obtainable according to Claim 10.

13. Freeze- or spray-dried preparations from which microparticulate systems as used according to Claim 1 are obtained by resuspension using a pharmaceutically suitable medium.

## Revendications

1. Utilisation de systèmes microparticulaires, composés d'un milieu de suspension pharmaceutiquement compatible, sans principe actif, et de microparticules, comprenant une enveloppe polymère biodégradable et un noyau contenant du gaz et un principe actif, pour préparer un médicament destiné à la libération in vivo du principe actif inclus, par ultrasons.

2. Utilisation de microparticules suivant la revendication 1, caractérisée en ce que la densité de la particule est inférieure à 0,8 g/cm³.

3. Utilisation de microparticules suivant la revendication 1, caractérisée en ce que la taille de particule est de 0,1-10 µm.

4. Utilisation de microparticules suivant la revendication 1, caractérisée en ce que sont utilisés comme polymère biodégradable des protéines, de la gélatine, du fibrinogène, du collagène ainsi que leurs dérivés, des polypeptides réticulés transversalement, des produits de réaction de protéines avec du polyéthylèneglycol, de l'amidon ou des dérivés d'amidon, de la chitine, du chitosane, de la pectine, du poly(acide lactique), des copolymères d'acide lactique et d'acide glycolique, des polycyanoacrylates, des polyesters, des polyamides, des polycarbonates, des polyphosphazènes, des poly(acides aminés), de la poly-(ε-caprolactone), ainsi que des copolymères d'acide lactique et de ε-caprolactone ou leurs mélanges.

5. Utilisation de microparticules suivant la revendication 1, caractérisée en ce qu'elles contiennent comme principe actif des toxines, des virus, des composants de virus, des composants de membranes cellulaires bactériologiques, des agents messagers solubles, des colorants, des composants de complément, des adjuvants, des agents trombolytiques, des facteurs nécrosants de tumeur, des acides nucléiques, des peptides, des protéines, des glycoprotéines, des hormones, des cytokines et/ou des prostaglandines.

6. Utilisation de microparticules suivant la revendication 1, caractérisée en ce qu'elles contiennent comme principe actif des cellules et/ou leurs composants.

7. Utilisation de microparticules suivant la revendication 1, caractérisée en ce qu'elles contiennent comme phase sous forme gazeuse, de l'air, de l'azote, de l'oxygène, du dioxyde de carbone, des gaz nobles, de l'ammoniac, des hydrocarbures halogénés ou partiellement halogénés, du SF₆ et/ou de la vapeur d'eau ou des liquides à bas point d'ébullition.

8. Utilisation de microparticules suivant la revendication 1, caractérisée en ce que la libération in vivo se fait par irradiation d'ultrasons diagnostiques.

9. Utilisation de microparticules suivant la revendication 1, caractérisée en ce que la libération in vivo se fait par irradiation d'un ultrason diagnostique d'une fréquence de 1-6 MHz, de préférence 1,5-5 MHz.

10. Procédé de préparation de microparticules contenant un principe actif, caractérisé en ce que des microparticules contenant du gaz sont traitées dans un autoclave avec une solution du principe actif dans un gaz supercritique, de préférence dans du dioxyde de carbone supercritique, de l'ammoniac supercritique ainsi que des gaz nobles supercritiques, puis lavées et lyophilisées.

11. Microparticule, contenant au moins un principe actif et une phase gazeuse, préparée suivant la revendication 10.

12. Microparticule, contenant une enveloppe de particule constituée d'au moins un polymère biodégradable, une phase gazeuse et au moins un acide nucléique, un peptide, une protéine, une glycoprotéine, une hormone et/ou une prostaglandine, pouvant être obtenue suivant la revendication 10.

13. Préparations lyophilisées ou séchées par pulvérisation, à partir desquelles on obtient par resuspension avec un milieu pharmaceutiquement compatible des systèmes microparticulaires, tels qu'ils sont utilisés suivant la revendication 1.
